(19) Europäisches Patentamt
European Patent Office
Office européen des brevets

(11) **EP 3 599 020 B1**

## (12) FASCICULE DE BREVET EUROPEEN

(45) Date de publication et mention
de la délivrance du brevet:
**07.04.2021 Bulletin 2021/14**

(21) Numéro de dépôt: **19186774.6**

(22) Date de dépôt: **17.07.2019**

(51) Int Cl.:
*B01J 21/04* (2006.01)　　　*B01J 23/04* (2006.01)
*B01J 35/10* (2006.01)　　　*B01J 23/92* (2006.01)
*B01J 20/08* (2006.01)　　　*B01J 37/08* (2006.01)
*C01F 7/44* (2006.01)　　　*C07C 1/24* (2006.01)
*C07C 5/25* (2006.01)　　　*C07C 11/06* (2006.01)

(54) **ALUMINE À ACIDITÉ ET STRUCTURE DE POROSITÉ OPTIMALES**

ALUMINIUMOXID MIT OPTIMALER AZIDITÄT UND PORÖSITÄTSSTRUKTUR

ALUMINA WITH OPTIMAL ACIDITY AND POROSITY STRUCTURE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **25.07.2018 FR 1856905**

(43) Date de publication de la demande:
**29.01.2020 Bulletin 2020/05**

(73) Titulaire: **AXENS
92508 Rueil-Malmaison Cedex (FR)**

(72) Inventeurs:
• **NEDEZ, Christophe
92508 RUEIL MALMAISON cedex (FR)**
• **DUCREUX, Olivier
92508 RUEIL MALMAISON cedex (FR)**

(74) Mandataire: **IFP Energies nouvelles
Département Propriété Industrielle
Rond Point de l'échangeur de Solaize
BP3
69360 Solaize (FR)**

(56) Documents cités:
**EP-A1- 0 692 306　　EP-B1- 3 074 368
JP-B2- 2 911 244**

• **P SOUZA SANTOS ET AL: "Standard transition
aluminas. Electron microscopy studies",
MATERIALS RESEARCH, vol. 3, no. 4, 1 octobre
2000 (2000-10-01) , pages 104-114, XP055334463,
BR ISSN: 1516-1439, DOI:
10.1590/S1516-14392000000400003**

EP 3 599 020 B1

**Description**

**DOMAINE DE L'INVENTION**

L'invention se rapporte au domaine de la catalyse de la déshydratation des alcools en oléfines par une alumine ayant une acidité et une structure de porosité optimales. Elle concerne plus particulièrement une alumine présentant une teneur en métaux alcalins réduite et une structure de porosité spécifique et son utilisation en tant que catalyseur notamment de la réaction de déshydratation des alcools, en particulier secondaires et tertiaires, ou en tant qu'adsorbant.

**CONTEXTE DE L'INVENTION ET ETAT DE LA TECHNIQUE**

Les alumines sont utilisées comme catalyseurs de nombreuses réactions chimiques et notamment de réactions nécessitant des conditions opératoires acides. C'est le cas, par exemple, des réactions de déshydratation des alcools. Dans de telles réactions, plus l'alumine sera active, plus la conversion sera élevée. Il existe donc un besoin pour des alumines présentant un caractère acide élevé.

Les alumines, en particulier acides, sont également souvent utilisées comme adsorbants.

Cependant, une alumine trop acide peut se révéler néfaste car des réactions secondaires peuvent survenir, d'où un rendement inférieur pour la réaction désirée. Dans la réaction de déshydratation des alcools, des réactions de condensation et de déshydrogénation peuvent avoir lieu. Ces réactions secondaires parasites font chuter le rendement et peuvent jouer un rôle néfaste sur la stabilité de l'alumine en diminuant, notamment, sa durée de vie. Par exemple, du coke peut se former, bloquant la porosité de l'adsorbant et conduisant à des durées de vie faibles.

De nombreux travaux ont été effectués afin de préparer des alumines optimisés en vue d'applications comme catalyseur en particulier de la réaction de déshydratation d'alcools ou comme adsorbant.

Le brevet FR 2 780 392 décrit une alumine activée avec une teneur en métaux alcalins, exprimée en $M_2O$, comprise entre 500 et 8 000 ppm en poids et une structure cristalline comprenant au moins 5% en poids d'alumine de phase η. Cette alumine est utilisée pour catalyser les réactions de déshydratation des alcools et d'isomérisation des oléfines pour lesquelles elle présente des performances satisfaisantes dans une gamme de teneurs en métaux alcalins élevée (supérieure à 2200 ppm poids), les performances étant plus limitées dans la gamme de teneurs en métaux alcalins faibles (moins de 1 500 ppm poids).

Le brevet US 9 145 342 décrit un procédé de production d'isobutène par déshydratation du alcool tert-butylique en phase gazeuse, en utilisant un catalyseur aluminique comprenant une teneur en Na, exprimée en $Na_2O$, comprise entre 0,1% et 0,6% poids (c'est-à-dire entre 1000 et 6000 ppm pds) et une teneur en Si, exprimée en $SiO_2$, inférieure ou égale à 0,4% poids et dont la structure de porosité est telle que volume poreux total est de 0,1 à 0,5 ml/g et le volume des pores de rayon supérieur à 70 Å est supérieur 60% du volume poreux total. Plus particulièrement, l'isobutène est produit avec un rendement satisfaisant lorsque la déshydratation de l'alcool ter-butylique est réalisée en présence d'un catalyseur aluminique contenant de la silice (au moins 0,06% poids de $SiO_2$), comprenant au moins 1300 ppm poids de $Na_2O$ et présentant un volume des pores de rayon d'au moins 70 Å correspondant à 74% et 78% du volume poreux total, lui-même aux alentours de 0,2 ml/g.

Dans le domaine de l'adsorption, le brevet EP 1 372 808 décrit un procédé d'élimination des molécules organiques oxygénées, telles que les alcools et acides organiques, présentes dans un effluent organique liquide ou gazeux, par adsorption sur des agglomérés d'alumine pouvant comprendre un composé dopant, tel qu'un métal alcalin, à une teneur massique, exprimée en $M_2O$, inférieure 5 000 ppm, et présentant une structure de porosité telle que le volume des pores dont le diamètre est supérieur à 70 Å est supérieur ou égal à 0,15 ml/g. Cet adsorbant capte les traces d'alcools et d'acide organiques présentes dans le flux à traiter en les adsorbant à température ambiante EP3074368B divulgue un procédé de production d'éthylène et propylène à partir d'un mélange d'alcools primaires éthanol et n-propanol, en présence d'une alumine. Dans les exemples, les catalyseurs C4, C5, C6, C7 et C8 comprennent une teneur en Na respectivement de 417, 394, 550, 448 et 401 ppm poids et une surface spécifique entre 152 et 270 m2/g.

L'objet de la présente invention est de proposer une alumine pouvant être utilisée comme catalyseur de la réaction de déshydratation des alcools, en particulier secondaires et tertiaires. En particulier, la présente invention propose une alumine permettant d'obtenir de bonnes performances en déshydratation d'alcools, voire meilleures que celles des alumines connues dans l'art antérieur.

**RESUME DE L'INVENTION**

La présente invention concerne une alumine selon la revendication 1 présentant une structure de porosité telle que le volume des pores dont le diamètre est compris entre 7 nm (70 Å) et 200 nm (2000 Å), déterminé par porosimétrie au mercure, est compris entre 0,15 et 0,50 ml/g, et comprenant au moins un métal alcalin (M), telle que la teneur massique en métal alcalin, exprimée en $M_2O$, est comprise entre 400 et 1500 ppm par rapport à la masse totale de l'alumine, présentant une surface spécifique BET d'au moins 150 m$^2$/g et contenant au plus 500 ppm poids de silice, exprimés en SiO$_2$, par rapport au poids total de ladite alumine.

De manière surprenante, la demanderesse a découvert que l'alumine selon la présente invention, présentant une teneur en métaux alcalins réduite et une structure poreuse particulière, notamment un volume de pores pour les pores de diamètre compris entre 7 nm (70 Å) et 200 nm (2000 Å) spécifique, entre 0,15 et 0,50 ml/g, permettait d'obtenir de bonnes performances en déshydratation d'alcools, meilleures que celles des alumines connues dans l'art antérieur. La demanderesse a en effet mis en évidence qu'une telle alumine permet d'obtenir une très bonne conversion des alcools en oléfines, une meilleure sélectivité et une stabilité améliorée, notamment en terme de durée de vie, tout en limitant les réactions secondaires parasites. Ainsi, la présente invention concerne également un procédé de transformation d'une charge comprenant au moins un alcool en effluent oléfinique, ledit procédé comprenant une étape de déshydratation dudit alcool dans laquelle l'alumine selon l'invention est mise en œuvre en tant que catalyseur. Avantageusement, l'alumine selon l'invention peut être mise en œuvre comme catalyseur de la réaction de déshydratation de tout type d'alcool, de préférence comprenant 3 à 9 atomes de carbone. L'alumine selon l'invention apparait particulièrement adaptée à la déshydratation des alcools secondaires et tertiaires, comprenant de façon très préférée de 3 à 6 atomes de carbone, pour produire les oléfines correspondantes.

## DESCRIPTION DE L'INVENTION

La présente invention concerne une alumine selon la revendication 1 présentant une structure de porosité telle que le volume des pores dont le diamètre est compris entre 7 nm (70 Å) et 200 nm (2000 Å), déterminé par porosimétrie au mercure, est compris entre 0,15 et 0,50 ml/g, et comprenant au moins un métal alcalin (M), telle que la teneur massique en métal alcalin, exprimée en M$_2$O, est comprise entre 400 et 1500 ppm par rapport à la masse totale de l'alumine, présentant une surface spécifique BET d'au moins 150 m2/g et contenant au plus 500 ppm poids de silice, exprimés en SiO2, par rapport au poids total de ladite alumine.

L'alumine selon l'invention présente une structure de porosité optimisée. Cette dernière est telle que le volume des pores dont le diamètre est compris entre 7 nm (70 Å) et 200 nm (2000 Å) (noté V$_{70Å}$-V$_{2000Å}$) est avantageusement supérieur ou égal à 0,15 ml/g, de préférence supérieur ou égal à 0,20 ml/g, et inférieur ou égal à 0,50 ml/g, de préférence inférieur ou égal à 0,40 ml/g.

Le volume des pores dont le diamètre est compris entre 7 nm (70 Å) et 200 nm (2000 Å) noté V$_{70Å}$-V$_{2000Å}$,

V$_{2000Å}$ représentant le volume créé par les pores de diamètre supérieur ou égal à 200 nm (2000 Å) et V$_{70Å}$ représentant le volume créé par les pores de diamètre supérieur ou égal à 7 nm (70 Å), correspond à la différence entre V$_{70Å}$ et V$_{2000Å}$. Il représente le volume créé par l'ensemble des pores de diamètre compris entre 7 nm (70 Å) et 200 nm (2000 Å). Les volumes V$_{70Å}$ et V$_{2000Å}$ sont mesurés sur un échantillon d'alumine par la méthode de porosimétrie au mercure selon la norme ASTM D4284-83 à une pression maximale de 4000 bar, utilisant une tension de surface de 484 dyne/cm et un angle de contact pour les supports alumine amorphe de 140°. L'alumine selon l'invention comprend au moins un métal alcalin, à une teneur avantageusement réduite. La teneur massique en métaux alcalins, exprimée en M$_2$O, de l'alumine selon l'invention est comprise entre 400 et 1500 ppm en poids, de préférence comprise entre 400 et 950 ppm poids par rapport à la masse totale de l'alumine.

Ledit métal alcalin de l'alumine selon l'invention est choisi parmi le sodium, le potassium, le lithium et leurs mélanges. Le métal alcalin préféré est le sodium. Le métal alcalin est généralement sous sa forme oxyde.

L'alumine selon l'invention présente ainsi une acidité réduite et une structure de porosité qui sont optimales en particulier pour la réaction de déshydratation des alcools. L'alumine selon l'invention présente également avantageusement une capacité d'isomérisation élevée. En particulier, l'alumine selon l'invention présente une capacité d'isomérisation de chaîne du 1-butène, à 300°C par rapport à l'équilibre thermodynamique, avantageusement supérieure strictement à 60%. En outre, la capacité d'isomérisation de chaîne du 1-butène par l'alumine selon l'invention, à 400°C par rapport à l'équilibre thermodynamique, est préférentiellement supérieure à 80 %.

De manière préférée, l'alumine selon l'invention a, en outre, un volume poreux total (VPT) supérieur ou égal à 0,50 ml/g.

Selon l'invention, le volume poreux total VPT est déterminé par calcul selon la formule suivante :

$$VPT = \frac{1}{Dg} - \frac{1}{Da}$$

dans laquelle (Dg) est la densité de grain et (Da) la densité absolue, mesurées par la méthode de picnométrie respectivement au mercure et à l'hélium.

Avantageusement, l'alumine présente un volume des pores de diamètre supérieur ou égal à 7 nm (70 Å), noté V$_{70Å}$, de préférence supérieur ou égal à 0,35 ml/g, préférentiellement supérieur ou égal à 0,45 ml/g, de manière préférée supérieur ou égal à 0,50 ml/g et de manière plus préférée encore supérieur ou égal à 0,52 ml/g. Le volume des pores de diamètre supérieur ou égal à 7 nm (70 Å), noté $V_{70Å}$, est avantageusement inférieur ou égal à 0,8 ml/g, de préférence inférieur ou égal à 0,70 ml/g.

L'alumine selon l'invention présente une surface spécifique BET d'au moins 150 $m^2$/g, de manière préférée d'au moins 200 $m^2$/g. Parallèlement, l'alumine selon l'invention présente avantageusement une surface spécifique BET d'au plus 350 $m^2$/g, de préférence d'au plus 325 $m^2$/g.

Cette surface spécifique est une surface mesurée par la méthode BET, c'est-à-dire la surface spécifique déterminée par adsorption d'azote conformément à la norme ASTM D 3663-78 établie à partir de la méthode BRUNAUER-EMMETT-TELLER décrite dans le périodique 'The Journal of the American Chemical Society", 6Q, 309 (1938).

Avantageusement, l'alumine selon l'invention contient au plus 500 ppm poids de silice, exprimés en $SiO_2$, de préférence au plus 100 ppm poids de silice par rapport au poids total de ladite alumine et plus préférentiellement est exempt de silice. De préférence, l'alumine selon l'invention contient de manière préférée au plus 800 ppm poids de soufre, de préférence au plus 500 ppm de soufre par rapport au poids total de ladite alumine. De manière préférée, l'alumine selon l'invention ne contient pas de phase active, ni d'agent(s) dopant(s), supplémentaire(s), autre(s) que les éléments définis précédemment, en particulier autre(s) que le métal alcalin défini ci-dessus.

La structure cristalline de l'alumine selon l'invention comprend avantageusement de l'alumine χ (chi) et de l'alumine amorphe. La structure cristalline de l'alumine selon l'invention est déterminée par diffraction des rayons X.

L'alumine selon l'invention contient de préférence strictement moins de 80 % poids d'alumine gamma, préférentiellement strictement moins de 50% poids d'alumine gamma et de manière préférée moins de 35% poids d'alumine gamma. L'alumine selon l'invention contient, en outre, avantageusement strictement moins de 35 % poids d'alumine η êta, de préférence strictement moins de 12% poids d'alumine η êta, préférentiellement strictement moins de 8% poids d'alumine η êta et encore plus préférentiellement strictement moins de 5% poids d'alumine η êta.

Avantageusement, l'alumine selon l'invention peut être obtenue par déshydratation rapide d'un hydroxyde ou oxyhydroxyde d'aluminium (hydrargillite, par exemple). Typiquement, l'alumine selon l'invention peut être obtenue au moyen du procédé de préparation décrit dans le brevet FR 2 527 197 par traitement dans un milieu aqueux ayant un pH inférieur à 9 d'une poudre d'alumine active, elle-même obtenue par déshydratation rapide d'hydrargillite dans un courant de gaz chauds, séchage par atomisation, puis calcination selon le procédé décrit dans le brevet EP 0 015 196. Elle peut également être obtenue de manière avantageuse au moyen du procédé de préparation décrit dans le brevet FR 2 449 650 par traitement d'une poudre d'alumine active présentant une structure mal cristallisée et/ou amorphe dans un milieu aqueux ayant un pH inférieur à 9, une alumine de structure mal cristallisée signifiant une alumine telle que l'analyse aux rayons X donne un diagramme ne présentant qu'une ou quelques raies diffuses correspondant aux phases cristallines des alumines de transition basse température, c'est-à-dire, essentiellement aux phases chi, rho, êta, gamma. L'alumine selon l'invention peut encore être obtenue par mélange d'au moins deux alumines, comme par exemple le mélange d'une alumine obtenue selon le procédé décrit dans le brevet FR 2 449 650 et d'une alumine obtenue selon le procédé décrit dans le brevet FR 2 527 197.

La déshydratation rapide d'hydroxydes ou d'oxyhydroxydes d'aluminium et plus particulièrement d'hydrargillite dans un courant de gaz chauds est réalisée typiquement dans n'importe quel appareillage approprié à l'aide d'un courant de gaz chauds, la température d'entrée des gaz dans l'appareillage variant généralement de 400 à 1200°C environ, le temps de contact de l'hydroxyde ou de l'oxyhydroxyde avec les gaz chauds étant généralement compris entre une fraction de seconde et 4-5 secondes. Ce procédé est décrit plus en détail dans la demande de brevet FR 1 108 011.

L'alumine obtenue par calcination rapide d'hydroxydes ou d'oxyhydroxydes d'aluminium conduit à une alumine ayant un taux élevé de $Na_2O$, de l'ordre de 3500 ppm. Après traitement en milieu aqueux à pH inférieur à 9, le taux de $Na_2O$ est considérablement abaissé. Le taux de $Na_2O$, après traitement en milieu aqueux à pH inférieur à 9, est généralement inférieur ou égal à 2 000 ppm poids, typiquement inférieur ou égal à 1 500 ppm poids. Avantageusement, après traitement en milieu aqueux à pH inférieur à 9, le taux de $Na_2O$ de l'alumine selon l'invention est entre 400 et 1 500 ppm poids, de préférence entre 400 et 950 ppm poids.

L'alumine selon l'invention pourra avantageusement se présenter sous toutes les formes habituelles connues, telles que sous forme de poudre, billes, matériaux extrudés et concassés. Les billes et les extrudés seront préférés. La taille des billes sera alors utilement comprise entre 0,5 et 10 mm, de préférence comprise entre 0,7 et 8 mm et préférentiellement comprise entre 0,8 et 5 mm. Les extrudés pourront être de forme cylindrique ou polylobée, pleins ou creux ; leur taille sera utilement comprise entre 0,5 et 5 mm, préférablement entre 0,7 et 3 mm.

Dans un mode de réalisation, l'alumine préparée peut être granulée, c'est-à-dire mise en forme, de préférence sous forme de billes. Les billes sont ensuite de préférence mûries à une température comprise entre 80 et 130°C. Les billes mûries sont enfin calcinées à une température comprise entre 400 et 1100°C.

A titre d'exemple, les billes peuvent être obtenues au moyen d'une technologie tournante, par agglomération d'une poudre d'alumine dans un drageoir ou un tambour. De manière connue, ce type de procédé permet d'obtenir des billes de diamètre et de répartitions de pores contrôlées, ces dimensions et ces répartitions étant, en général, créées pendant l'étape d'agglomération. La porosité peut être créée par différents moyens comme le choix de la granulométrie de la poudre d'alumine ou l'agglomération de plusieurs poudres d'alumine de différentes granulométries. Une autre méthode consiste à mélanger à la poudre d'alumine, avant ou pendant l'étape d'agglomération, un composé, appelé porogène, disparaissant par chauffage et créant ainsi une porosité dans les billes. Comme composés porogènes utilisés, on peut citer, à titre d'exemple, la farine de bois, le charbon de bois, le soufre, des goudrons, des matières plastiques ou émulsions de matières plastiques telles que le polychlorure de vinyle, des alcools polyvinyliques, la naphtaline ou analogues. La quantité de composés porogènes ajoutés est déterminée par le volume désiré. Un ou plusieurs traitements thermiques viennent alors achever la mise en forme des billes.

Dans un autre mode de réalisation, l'alumine préparée peut être sous forme d'extrudés, obtenus par exemple par malaxage puis extrusion d'un gel d'alumine, ou d'une poudre d'alumine, ou d'un mélange de matières premières différentes.

La présente invention concerne également un procédé de transformation d'une charge comprenant au moins un alcool, en effluent oléfinique, ledit procédé comprenant une étape de déshydratation dudit alcool en présence de l'alumine selon l'invention. Avantageusement, l'alumine selon l'invention permet d'atteindre un taux de conversion de l'alcool, en particulier d'alcool secondaire et/ou tertiaire, en oléfine élevé, notamment supérieur ou égal à 30%, de préférence supérieur ou égal à 50%, préférentiellement supérieur ou égal à 65% et de façon encore plus préférée supérieur ou égal à 70%.

Selon l'invention, par effluent oléfinique, on entend un effluent comprenant, plus de 25% poids d'oléfines, de préférence plus de 50% poids d'oléfines, préférentiellement au moins 60% poids d'oléfines, plus préférentiellement au moins 75% poids d'oléfines et de façon encore plus préférée au moins 90% poids d'oléfines par rapport au poids dudit effluent, de préférence d'oléfines correspondantes (ou visées), c'est-à-dire ayant le même nombre d'atomes de carbone que l'alcool compris dans la charge.

La charge à traiter comprend avantageusement au moins un alcool, notamment au moins 50% poids d'alcool, de préférence au moins 70% poids, préférentiellement au moins 80% poids, plus préférentiellement au moins 85% poids et de manière plus préférée encore au moins 90% poids dudit alcool par rapport au poids de ladite charge. La charge peut comprendre un alcool ou un mélange d'alcools. Les alcools composant la charge peuvent être produit à partir de produits pétroliers ou de produits chimiques ou bien peuvent provenir de la biomasse.

L'alumine selon l'invention est mise en œuvre comme catalyseur de déshydratation de tout type d'alcool pour produire l'oléfine correspondante, c'est-à-dire ayant le même nombre d'atomes de carbone. Les alcools ayant de 3 à 9 atomes de carbone, préférentiellement de 4 à 6 atomes de carbone, en particulier 4 atomes de carbone, sont préférés.

L'alumine selon l'invention est avantageusement mise en œuvre comme catalyseur de la déshydratation des alcools secondaires et/ou tertiaires, en particulier des alcools tertiaires. Selon un mode de réalisation de l'invention, la charge comprend un alcool secondaire choisi parmi : isopropanol, 2-butanol, 3-methylbutan-2-ol, pentan2-ol, pentan-3-ol, et leurs mélanges. Selon un autre mode de réalisation de l'invention, la charge comprend un alcool tertiaire choisi parmi : 2-methylpropan-2-ol (tert-butanol ou alcohol *tert*-butylique, « tertiary butyl alcohol » dans la terminologie anglo-saxonne noté TBA) et/ou du 2-methylbutan-2-ol (tertiary pentanol *tert*-Amyl alcohol noté TAA dans la terminologie anglo-saxonne). De préférence, l'alumine selon l'invention est mise en œuvre pour catalyser la déshydratation du 2- méthylpropan-2-ol (tert-butanol) en isobutène et/ou la déshydratation du 2-méthylbutan-2-ol (ou «tert-pentanol) en 2 méthyl-but-2-ène et/ou 2 méthyl-but-1-ène. L'étape de déshydratation comprend une section réactionnelle comprenant un ou plusieurs réacteurs comprenant le catalyseur. Le (ou les) réacteur(s) peut(peuvent) être un(des) réacteur(s) discontinu(s) ou un(des) réacteur(s) à lit fixe continu(s) (radial, isotherme, adiabatique, etc.). Dans un mode préféré, la déshydratation se déroule en continu dans une configuration de réacteur à lit fixe en utilisant plusieurs réacteurs en série de tailles égales ou différentes et/ou plusieurs réacteurs « permutables » opérés en parallèle. Avantageusement, la réaction de déshydratation est mise en œuvre dans au moins deux réacteurs distincts, de préférence entre 2 et 6 réacteurs distincts, notamment dans 4 réacteurs distincts, de préférence en série, l'effluent obtenu en sortie d'un réacteur pouvant être avantageusement réchauffé avant d'être envoyé dans le réacteur suivant.

La réaction de déshydratation est mise en œuvre, dans la section réactionnelle, dans les conditions connues de l'homme du métier, typiquement en phase gaz, en phase liquide ou en phase mixte (c'est-à-dire un mélange de phases gaz et liquide), de préférence en phase gaz, à une température comprise entre 200°C et 450°C, de préférence entre 225°C et 410°C, à une pression comprise entre 0,1 et 4 MPa, de préférence entre 0,5 et 2 MPa, et avec une vitesse volumique horaire (VVH) comprise entre 0,5 $h^{-1}$ et 10 $h^{-1}$, de préférence entre 2 et 7 $h^{-1}$.

Par vitesse volumique horaire (VVH), on entend le rapport entre le débit volumique de la charge en entrée de réacteur en $m^3$/h à 15°C, 1 atm divisé par le volume de catalyseur en $m^3$ contenu dans le réacteur.

Selon un mode préféré de l'invention, le procédé est un procédé de transformation d'une charge comprenant au moins un alcool secondaire, un alcool tertiaire ou leur mélange, en un effluent oléfinique, ledit procédé comprenant une étape de déshydratation dudit alcool qui comprend une section réactionnelle mise en œuvre en présence de l'alumine selon l'invention, en phase gaz, en phase liquide ou en phase mixte, préférentiellement en phase gazeuse, à une température comprise entre 200°C et 450°C, à une pression comprise entre 0,1 et 4 MPa et avec une vitesse volumique horaire (VVH) comprise entre 0,5 $h^{-1}$ et 10 $h^{-1}$. La pression absolue en entrée de section réactionnelle est avantageusement choisie de manière à ce que la charge soit en phase gazeuse à l'entrée du réacteur.

Selon un mode très particulier de l'invention, le procédé est un procédé de transformation d'une charge comprenant au moins un alcool secondaire, comprenant avantageusement 4 atomes de carbone, en un effluent oléfinique, ledit procédé comprenant une étape de déshydratation dudit alcool qui comprend une section réactionnelle mise en œuvre en présence de l'alumine selon l'invention, préférentiellement en phase gazeuse, à une température comprise entre 350°C et 450°C, plus préférentiellement comprise entre 350°C et 400°C, à une pression comprise préférentiellement entre 0,5 et 1,2 MPa, et avec une vitesse volumique horaire (VVH) comprise entre 2 $h^{-1}$ et 7 $h^{-1}$. La pression absolue en entrée de section réactionnelle est avantageusement choisie de manière à ce que la charge soit en phase gazeuse à l'entrée du réacteur.

Selon un autre mode très particulier de l'invention, le procédé est un procédé de transformation d'une charge comprenant au moins un alcool tertiaire, comprenant avantageusement 4 atomes de carbone, en un effluent oléfinique, ledit procédé comprenant une étape de déshydratation dudit alcool qui comprend une section réactionnelle mise en œuvre en présence de l'alumine selon l'invention, préférentiellement en phase gazeuse, à une température comprise entre 250°C et 410°C, plus préférentiellement comprise entre 275°C et 380°C, à une pression comprise préférentiellement entre 0,5 et 1,7 MPa, et avec une vitesse volumique horaire (VVH) comprise entre 2 $h^{-1}$ et 4 $h^{-1}$. La pression absolue en entrée de section réactionnelle est avantageusement choisie de manière à ce que la charge soit en phase gazeuse à l'entrée du réacteur.

Avantageusement, l'alumine utilisée comme catalyseur dans la présente invention peut être régénérée, notamment plusieurs fois, par exemple en procédant au brûlage contrôlé du coke déposé au cours de la réaction sur l'alumine. Ainsi, le procédé de transformation selon l'invention peut avantageusement être effectué en continu dans plusieurs réacteurs "permutables" parallèles dans lesquels, lorsqu'un ou plusieurs réacteurs fonctionnent, un autre subit une régénération du catalyseur.

Le procédé selon l'invention peut éventuellement comprendre une étape de purification de la charge, en amont de l'étape de déshydratation comprenant la section réactionnelle. Cette étape éventuelle de purification, comprenant au moins une section de purification, permet d'éliminer les impuretés, par exemples les impuretés organiques éventuellement azotées ou soufrées, susceptibles d'affecter le catalyseur de ladite section réactionnelle. Toute méthode de purification connue de l'Homme du métier peut être utilisée dans ladite (ou lesdites) section(s) de purification.

Avantageusement, le procédé selon l'invention peut typiquement comprendre en outre une ou plusieurs autres sections, comme une section de chauffage pour chauffer la charge à traiter avant son entrée dans la section réactionnelle, une section de séparation pour séparer l'effluent en sortie de section réactionnelle et récupérer les différents produits obtenus, une section de purification pour récupérer un effluent oléfinique purifié, en particulier comprenant au moins 90% poids d'oléfines visées.

L'alumine selon l'invention peut également être utilisée comme adsorbant.

L'invention sera mieux comprise au vu des exemples suivants, présentés à titre illustratif et non limitatif.

## METHODES DE MESURE

### Méthode de porosimétrie au mercure

Le volume des pores de diamètre supérieur ou égale à 7 nm (70 Å) ($V_{70Å}$) et le volume des pores de diamètre supérieur ou égale à 200 nm (2000 Å) ($V_{2000Å}$) sont mesurés par la méthode de porosimétrie au mercure selon la norme ASTM D4284-83 à une pression maximale de 4000 bar, utilisant une tension de surface de 484 dyne/cm et un angle de contact pour les supports alumine amorphe de 140°, sur un échantillon d'alumine.

La loi de Kelvin donne la relation entre la pression, le diamètre du plus petit pore dans lequel le mercure pénètre à ladite pression, l'angle de mouillage et la tension superficielle selon la formule : $Ø = (-4\gamma \cos\theta) / P$

dans laquelle

- Ø représente le diamètre du pore,

- $\gamma$ la tension superficielle du mercure,

- $\theta$ l'angle de contact entre le mercure et le solide

- P la pression absolue.

En pratique, l'échantillon d'alumine est placé dans une colonne, dans laquelle on introduit du mercure sous une pression P. Le mercure ne mouillant pas l'alumine, sa pénétration ou sa non-pénétration dans les pores de l'échantillon ayant un diamètre donné est fonction de la valeur de P. Les pores les plus fins nécessitent, pour être remplis, l'établissement d'une pression P plus élevée que pour le remplissage des pores plus grossiers. La mesure de la quantité de mercure pénétrant dans l'échantillon pour différentes valeurs de P permet de connaître le volume occupé par les pores de diamètre supérieur à des valeurs données de ce diamètre.

**Mesure des capacités d'isomérisation de chaîne du 1-butène :**

On introduit 500 mg d'alumine selon l'invention broyée (taille des particules comprise entre 400 et 500 $\mu$m) dans un réacteur en verre. Le produit est conditionné in situ pendant 2 h à 300°C sous un balayage d'hélium présentant un débit de 3,9 l/h. La température du réacteur est ensuite augmentée en passant par deux paliers à 300 puis 400°C. A chaque palier de température, trois injections de 0,2 ml du butène sont faites dans le réacteur, toujours maintenu sous un balayage d'hélium de 3,9 l/h.

L'analyse des gaz de sortie se fait par chromatographie en phase gazeuse. Elle permet de mesurer la quantité de 1-butène non transformé, ainsi que la quantité de 2-cis-et 2-trans-butène formés.

On détermine par le calcul la constante d'équilibre thermodynamique Kth(T) théorique et, par le résultat des mesures, la constante d'équilibre K(T) réelle.

$$Kth(T) = \frac{[2 - cisbutène]e \; + \; [2 - trans - butène]e}{[1 - butène]e \; + \; [2 - cis - butène]e \; + \; [2 - trans - butène]e}$$

$$K(T) = \frac{[2 - cisbutène] \; + \; [2 - trans - butène]}{[1 - butène] \; + \; [2 - cis - butène] \; + \; [2 - trans - butène]}$$

dans lesquelles T est la température du butène à la sortie du réacteur. Les autres valeurs représentent les concentrations en sortie de réacteur ou à l'équilibre ([ ]e) pour ladite température T. Le pouvoir isomérisant ou taux d'isomérisation A(T) est donné par la formule suivante:

$$A(T) \; = \frac{K(T) \; X \; 100}{Kth(T)}$$

**EXEMPLES**

### Exemple 1

Chacune des alumines A à I (cf. Tableau 1) est préparée de la façon suivante :

De la poudre d'hydrargillite subit une déshydratation rapide à 1000°C, à l'aide d'un courant de gaz chaud pour obtenir une poudre 1, contentant 3200 ppm poids de $Na_2O$. Une partie de la poudre 1 est traitée en milieu aqueux pour donner une poudre 2 dont le taux de $Na_2O$ est de 500 ppm en poids. Un mélange des poudres 1 et 2 est effectué. Le mélange est ensuite granulé sous forme de billes avec de l'eau, en présence éventuellement d'un porogène, pour obtenir une alumine sous forme de bille et présentant un taux de sodium dépendant du ratio poudre 1 sur poudre 2.

Les billes sont mûries puis calcinées.

Les caractéristiques des alumines A à I préparées sont données dans le Tableau 1. De plus, les alumines A à I obtenues contiennent moins de 500 ppm massique de silice.

### Exemple 2

Les capacités d'isomérisation du 1-butène sont déterminées pour chacune des alumines A à I préparées selon l'Exemple 1, conformément à la méthode et les conditions opératoires décrites ci-avant.

Les résultats sont donnés dans le Tableau 1. Ils correspondent à la moyenne des trois injections de butène effectuées à 300°C et à la moyenne des trois injections effectuées à 400°C.

Tableau 1

| Alumine | Teneur en $Na_2O$ | $V_{70Å}$ | $V_{2000Å}$ | $V_{70Å}-V_{2000Å}$ | Surface Spécifique SBET | Volume poreux total (VPT) | Taux d'isomérisation à (T) | |
|---|---|---|---|---|---|---|---|---|
| | ppm poids | ml/g | ml/g | ml/g | $m^2/g$ | ml/g | (T=300°C) % | (T=400°C) % |
| A | 700 | 0,398 | 0,128 | 0,270 | 270 | 0,537 | 93,3 | 94,8 |
| B | 700 | 0,557 | 0,207 | 0,350 | 260 | 0,655 | 92,5 | 94,0 |
| C | 500 | 0,548 | 0,187 | 0,361 | 260 | 0,585 | 92,7 | 94,2 |
| D | 500 | 0,625 | 0,170 | 0,455 | 180 | 0,680 | 46,3 | 92,2 |
| E | 2 000 | 0,333 | 0,180 | 0,153 | 360 | 0,555 | 9,0 | 55,6 |
| F | 2 000 | 0,169 | 0,039 | 0,130 | 350 | 0,445 | 19,6 | 70,6 |
| G | 3 200 | 0,234 | 0,053 | 0,181 | 335 | 0,441 | 1,7 | 26,6 |
| H | 500 | 0,744 | 0,047 | 0,697 | 105 | 0,752 | 9,5 | 57,3 |
| I | 500 | 0,534 | 0,410 | 0,124 | 7 | 0,535 | 0,5 | 7,6 |

La capacité d'isomérisation des alumines A à D selon l'invention est supérieure à celle des alumines E à I non conformes à l'invention.

**Exemple 3**

Tests de déshydratation de l'isopropanol en présence des alumines A à I, dans des réacteurs à l'échelle pilote.

Chacune des alumines A à I sont testées de la façon suivante :

Un échantillon d'alumine est broyé pour obtenir une poudre de granulométrie entre 0,8 et 1 mm. Un réacteur pilote est rempli par 0,4 g de d'alumine broyée. Dans le réacteur, l'alumine broyée est chauffée durant 2 heures à 400°C. Après retour à la température de l'expérience (T=225°C), l'échantillon d'alumine est soumis à douze injections consécutives d'un flux constitué d'azote ayant traversé un bain d'isopropanol maintenu à 30°C, avec un débit de 4 l/h. Les flux entrant et sortant de chacune des injections sont analysés par chromatographie en phase gazeuse.

Les résultats donnés dans le Tableau 2 correspondent à la mesure effectuée après la douzième injection.

Il apparait que le produit principal de la réaction est le propylène, correspondant au produit de la réaction de déshydratation due l'isopropanol. Les analyses chromatographiques montrent également que le diisopropyl-éther, produit de la réaction secondaire de condensation, et l'acétone, produit de la réaction secondaire de la déshydrogénation sont en quantités faibles.

Tableau 2 : Conversion de l'isopropanol et sélectivité en propylène obtenues à 225°C en présence des alumines A à I

| | | A | B | C | D | E | F | G | H | I |
|---|---|---|---|---|---|---|---|---|---|---|
| Conversion Isopropanol | % | 67 | 73 | 72 | 30 | 12 | 21 | 3 | 9 | <1 |
| Sélectivité en propylène | % | 97 | 98 | 98 | 75 | ns | 50 | ns | ns | ns |
| ns = non significatif | | | | | | | | | | |

En présence des alumines A à D selon l'invention, les taux de conversion sont très satisfaisants et les sélectivités en propylène sont élevés. Les performances catalytiques obtenues des alumines A à D conformes à l'invention obtenues lors de la réaction de déshydratation d'un alcool, comme l'isopropanol, sont meilleures que celles obtenues avec les alumines E à I non conformes.

**EP 3 599 020 B1**

**Revendications**

1. Alumine présentant une structure de porosité telle que le volume des pores dont le diamètre est compris entre 7 nm (70 Å) et 200 nm (2000 Å), déterminé par porosimétrie au mercure, est compris entre 0,15 et 0,50 ml/g, comprenant au moins un métal alcalin (M), telle que la teneur massique en métal alcalin, exprimée en $M_2O$, est comprise entre 400 et 1500 ppm par rapport à la masse totale de l'alumine, présentant une surface spécifique BET d'au moins 150 $m^2/g$ et contenant au plus 500 ppm poids de silice, exprimés en $SiO_2$, par rapport au poids total de ladite alumine.

2. Alumine selon la revendication 1, dans laquelle le volume des pores dont le diamètre est compris entre 7 nm (70 Å) et 200 nm (2000 Å) est supérieur ou égal à 0,20 ml/g, et de préférence inférieur ou égal à 0,40 ml/g.

3. Alumine selon la revendication 1 ou 2, dans laquelle la teneur massique en métal alcalin, exprimée en $M_2O$, est comprise entre 400 et 950 ppm par rapport à la masse totale de l'alumine.

4. Alumine selon l'une des revendications précédentes, dans laquelle le volume poreux total est supérieur ou égal à 0,50 ml/g.

5. Alumine selon l'une des revendications précédentes, présentant un volume des pores de diamètre supérieur ou égal à 7 nm (70 Å), noté $V_{70Å}$, supérieur ou égal à 0,35 ml/g, préférentiellement supérieur ou égal à 0,45 ml/g, de manière préférée supérieur ou égal à 0,50 ml/g et de manière plus préférée encore supérieur ou égal à 0,52 ml/g.

6. Alumine selon l'une des revendications précédentes, présentant une surface spécifique BET d'au moins 200 $m^2/g$, et d'au plus 350 $m^2/g$, de préférence d'au plus 325 $m^2/g$.

7. Alumine selon l'une des revendications précédentes, contenant au plus 100 ppm poids de silice, exprimés en $SiO_2$, par rapport au poids total de ladite alumine et plus préférentiellement étant exempt de silice.

8. Alumine selon l'une des revendications précédentes, ayant une structure comprenant de l'alumine $\chi$ (chi) et de l'alumine amorphe.

9. Alumine selon l'une des revendications précédentes, contenant strictement moins de 80% d'alumine gamma, préférentiellement strictement moins de 50% poids d'alumine gamma et de manière préférée moins de 35% poids d'alumine gamma.

10. Alumine selon l'une des revendications précédentes, contenant strictement moins de 35 % poids d'alumine $\eta$ êta, de préférence strictement moins de 12% poids d'alumine $\eta$ êta, préférentiellement strictement moins de 8% poids d'alumine $\eta$ êta et encore plus préférentiellement strictement moins de 5% poids d'alumine $\eta$ êta.

11. Alumine selon l'une des revendications précédentes obtenue par déshydratation d'un hydroxyde ou oxyhydroxyde d'aluminium dans un appareillage et sous courant de gaz à une température d'entrée des gaz dans l'appareillage variant de 400 à 1200°C.

12. Procédé de transformation d'une charge comprenant au moins un alcool, en effluent oléfinique, ledit procédé comprenant une étape de déshydratation dudit alcool en présence de l'alumine selon l'une des revendications 1 à 11.

13. Procédé selon la revendication précédente, dans lequel la charge comprend au moins 50% poids d'alcool, de préférence au moins 70% poids, préférentiellement au moins 80% poids, plus préférentiellement au moins 85% poids et de manière plus préférée encore au moins 90% poids dudit alcool par rapport au poids de ladite charge.

14. Procédé selon la revendication 12 ou 13, dans lequel ledit alcool comprend de 3 à 9 atomes de carbone, préférentiellement de 4 à 6 atomes de carbone, encore plus préférentiellement 4 atomes de carbone.

15. Procédé selon l'une des revendications 12 à 14, dans lequel ledit alcool est un alcool secondaire et/ou tertiaire, de préférence tertiaire.

16. Procédé selon l'une des revendications 12 à 15, dans lequel l'étape de déshydratation comprend une section réactionnelle dans laquelle la réaction de déshydratation est mise en œuvre en phase gaz, en phase liquide ou en phase mixte, à une température comprise entre 200°C et 450°C, à une pression comprise entre 0,1 et 4 MPa et avec une vitesse volumique horaire (VVH) comprise entre 0,5 h$^{-1}$ et 10 h$^{-1}$.

**Patentansprüche**

1. Aluminiumoxid, das eine Struktur mit einer derartigen Porosität, dass das durch Quecksilberporosimetrie bestimmte Volumen der Poren mit einem Durchmesser zwischen 7 nm (70 Å) und 200 nm (2000 Å) zwischen 0,15 und 0,50 ml/g liegt, aufweist, mindestens ein Alkalimetall (M) derart umfasst, dass der Massengehalt an Alkalimetall, ausgedrückt als $M_2O$, zwischen 400 und 1500 ppm, bezogen auf die Gesamtmasse des Aluminiumoxids, liegt, eine spezifische BET-Oberfläche von mindestens 150 m$^2$/g aufweist und höchstens 500 Gew.-ppm Siliciumdioxid, ausgedrückt als $SiO_2$, bezogen auf das Gesamtgewicht des Aluminiumoxids, enthält.

2. Aluminiumoxid nach Anspruch 1, wobei das durch Quecksilberporosimetrie bestimmte Volumen der Poren mit einem Durchmesser zwischen 7 nm (70 Å) und 200 nm (2000 Å) größer oder gleich 0,20 ml/g und bevorzugt kleiner oder gleich 0,40 ml/g ist.

3. Aluminiumoxid nach Anspruch 1 oder 2, wobei der Massengehalt an Alkalimetall, ausgedrückt als $M_2O$, zwischen 400 und 950 ppm, bezogen auf die Gesamtmasse des Aluminiumoxids, liegt.

4. Aluminiumoxid nach einem der vorhergehenden Ansprüche, wobei das Gesamtporenvolumen größer oder gleich 0,50 ml/g ist.

5. Aluminiumoxid nach einem der vorhergehenden Ansprüche mit einem Volumen der Poren mit einem Durchmesser größer oder gleich 7 nm (70 Å), das als $V_{70Å}$ bezeichnet wird, größer oder gleich 0,35 ml/g, bevorzugt größer oder gleich 0,45 ml/g, vorzugsweise größer oder gleich 0,50 ml/g und noch weiter bevorzugt größer oder gleich 0,52 ml/g.

6. Aluminiumoxid nach einem der vorhergehenden Ansprüche mit einer spezifischen BET-Oberfläche von mindestens 200 m$^2$/g und höchstens 350 m$^2$/g, bevorzugt höchstens 325 m$^2$/g.

7. Aluminiumoxid nach einem der vorhergehenden Ansprüche, das höchstens 100 Gew.-ppm Siliciumdioxid, ausgedrückt als $SiO_2$, bezogen auf das Gesamtgewicht des Aluminiumoxids, enthält und weiter bevorzugt frei von Siliciumdioxid ist.

8. Aluminiumoxid nach einem der vorhergehenden Ansprüche mit einer Struktur, die $\chi$-Aluminiumoxid (chi-Aluminiumoxid) und amorphes Aluminiumoxid umfasst.

9. Aluminiumoxid nach einem der vorhergehenden Ansprüche, das streng weniger als 80 Gew.-% gamma-Aluminiumoxid, bevorzugt streng weniger als 50 Gew.-% gamma-Aluminiumoxid und vorzugsweise weniger als 35 Gew.-% gamma-Aluminiumoxid enthält.

10. Aluminiumoxid nach einem der vorhergehenden Ansprüche, das streng weniger als 35 Gew.-% $\eta$-Aluminiumoxid (eta-Aluminiumoxid), bevorzugt streng weniger als 12 Gew.-% $\eta$-Aluminiumoxid, bevorzugt streng weniger als 8 Gew.-% $\eta$-Aluminiumoxid und noch weiter bevorzugt streng weniger als 5 Gew.-% $\eta$-Aluminiumoxid enthält.

11. Aluminiumoxid nach einem der vorhergehenden Ansprüche, erhalten durch Dehydratisierung eines Aluminiumhydroxids oder -oxidhydroxids in einer Apparatur und unter einem Gasstrom mit einer Eintrittstemperatur der Gase in die Apparatur im Bereich von 400 bis 1200 °C.

12. Verfahren zur Umwandlung eines Einsatzstoffs, der mindestens einen Alkohol umfasst, in einen olefinischen Austragsstrom, wobei das Verfahren einen Schritt der Dehydratisierung des Alkohols in Gegenwart des Aluminiumoxids nach einem der Ansprüche 1 bis 11 umfasst.

13. Verfahren nach dem vorhergehenden Anspruch, wobei der Einsatzstoff mindestens 50 Gew.-% Alkohol, bevorzugt mindestens 70 Gew.-%, vorzugsweise mindestens 80 Gew.-%, weiter bevorzugt mindestens 85 Gew.-% und noch weiter bevorzugt mindestens 90 Gew.-% des Alkohols, bezogen auf das Gewicht des Einsatzstoffs, umfasst.

14. Verfahren nach Anspruch 12 oder 13, wobei der Alkohol 3 bis 9 Kohlenstoffatome, bevorzugt 4 bis 6 Kohlenstoffatome und noch weiter bevorzugt 4 Kohlenstoffatome umfasst.

**15.** Verfahren nach einem der Ansprüche 12 bis 14, wobei es sich bei dem Alkohol um einen sekundären und/oder tertiären Alkohol, vorzugsweise einen tertiären Alkohol, handelt.

**16.** Verfahren nach einem der Ansprüche 12 bis 15, wobei der Dehydratisierungsschritt einen Reaktionsabschnitt umfasst, in dem die Dehydratisierungsreaktion in der Gasphase, in der Flüssigphase oder in einer Mischphase bei der Temperatur zwischen 200 °C und 450 °C, bei einem Druck zwischen 0,1 und 4 MPa und mit einer Katalysatorbelastung (WH) zwischen 0,5 $h^{-1}$ und 10 $h^{-1}$ durchgeführt wird.

**Claims**

**1.** Alumina exhibiting a structure with a porosity such that the volume of the pores having a diameter of between 7 nm (70 Å) and 200 nm (2000 Å), determined by mercury porosimetry, is between 0.15 and 0.50 ml/g, comprising at least one alkali metal (M), such that the content by weight of alkali metal, expressed as $M_2O$, is between 400 and 1500 ppm with respect to the total weight of the alumina, exhibiting a BET specific surface area of at least 150 $m^2$/g and containing at most 500 ppm by weight of silica, expressed as $SiO_2$, with respect to the total weight of said alumina.

**2.** Alumina according to Claim 1, in which the volume of the pores having a diameter of between 7 nm (70 Å) and 200 nm (2000 Å) is greater than or equal to 0.20 ml/g, and preferably less than or equal to 0.40 ml/g.

**3.** Alumina according to Claim 1 or 2, in which the content by weight of alkali metal, expressed as $M_2O$, is between 400 and 950 ppm with respect to the total weight of the alumina.

**4.** Alumina according to one of the preceding claims, in which the total pore volume is greater than or equal to 0.50 ml/g.

**5.** Alumina according to one of the preceding claims, exhibiting a volume of the pores with a diameter of greater than or equal to 7 nm (70 Å), denoted $V_{70Å}$, of greater than or equal to 0.35 ml/g, preferentially of greater than or equal to 0.45 ml/g, preferably of greater than or equal to 0.50 ml/g and more preferably still of greater than or equal to 0.52 ml/g.

**6.** Alumina according to one of the preceding claims, exhibiting a BET specific surface area of at least 200 $m^2$/g, and of at most 350 $m^2$/g, preferably of at most 325 $m^2$/g.

**7.** Alumina according to one of the preceding claims, containing at most 100 ppm by weight of silica, expressed as $SiO_2$, with respect to the total weight of said alumina, and more preferably being devoid of silica.

**8.** Alumina according to one of the preceding claims, having a structure comprising χ-alumina and amorphous alumina.

**9.** Alumina according to one of the preceding claims, containing strictly less than 80% of γ-alumina, preferentially strictly less than 50% by weight of γ-alumina and preferably less than 35% by weight of γ-alumina.

**10.** Alumina according to one of the preceding claims, containing strictly less than 35% by weight of η-alumina, preferably strictly less than 12% by weight of η-alumina, preferentially strictly less than 8% by weight of η-alumina and more preferentially still strictly less than 5% by weight of η-alumina.

**11.** Alumina according to one of the preceding claims, obtained by dehydration of an aluminium hydroxide or oxyhydroxide in an appliance under a stream of gas at an inlet temperature of the gases in the appliance varying from 400 to 1200°C.

**12.** Process for the transformation of a feedstock comprising at least one alcohol into an olefinic effluent, said process comprising a step of dehydration of said alcohol in the presence of the alumina according to one of Claims 1 to 11.

**13.** Process according to the preceding claim, in which the feedstock comprises at least 50% by weight of alcohol, preferably at least 70% by weight, preferentially at least 80% by weight, more preferably at least 85% by weight and more preferably still at least 90% by weight of said alcohol, with respect to the weight of said feedstock.

**14.** Process according to Claim 12 or 13, in which said alcohol comprises from 3 to 9 carbon atoms, preferentially from 4 to 6 carbon atoms and more preferentially still 4 carbon atoms.

**15.** Process according to one of Claims 12 to 14, in which said alcohol is a secondary and/or tertiary alcohol, preferably a tertiary alcohol.

**16.** Process according to one of Claims 12 to 15, in which the dehydration step comprises a reaction section in which the dehydration reaction is carried out in the gas phase, in the liquid phase or in a mixed phase, at a temperature of between 200°C and 450°C, at a pressure of between 0.1 and 4 MPa and with an hourly space velocity (HSV) of between $0.5\ h^{-1}$ and $10\ h^{-1}$.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2780392
- US 9145342 B
- EP 1372808 A
- EP 3074368 B
- FR 2527197
- EP 0015196 A
- FR 2449650
- FR 1108011

**Littérature non-brevet citée dans la description**

- *The Journal of the American Chemical Society,* 1938, vol. 6Q, 309